# EUROPÄISCHE PATENTSCHRIFT

(11) **EP 3 897 470 B1**
(45) Veröffentlichungstag und Bekanntmachung des Hinweises auf die Patenterteilung: **11.09.2024**
(21) Anmeldenummer: 19817220.7
(22) Anmeldetag: 04.12.2019
(51) Int. Cl.: A61F 2/78, A61F 5/01, A61F 2/72

(54) **ELASTISCHES FLÄCHENMATERIAL, BANDAGE DARAUS UND VERFAHREN ZU DESSEN HERSTELLUNG**
ELASTIC SHEET MATERIAL, BANDAGE MADE THEREOF AND METHOD FOR PRODUCING SAID MATERIAL
MATÉRIAU EN FEUILLE ÉLASTIQUE, BANDAGE FABRIQUÉ À PARTIR DE CELUI-CI ET SON PROCÉDÉ DE FABRICATION

(30) Priorität: 18.12.2018 DE 102018132640
(43) Veröffentlichungstag der Anmeldung: 27.10.2021
(73) Patentinhaber: Ottobock SE & Co. KGaA, 37115 Duderstadt (DE)
(72) Erfinder: HORMES, Helle, 37154 Northeim (DE); SCHULZ, Christoph, 37115 Gerblingerode (DE); JUNG, Marcel, 37115 Duderstadt (DE); KROLL-ORYWAHL, Olaf, 37154 Northeim (DE); KOPPE, Mario, 37085 Göttingen (DE); LÜBBERS, Christopher, Lee, 37085 Göttingen (DE); MÜLLER, Hans-Peter, 95145 Oberkotzau (DE)
(74) Vertreter: Gramm, Lins & Partner Patent- und Rechtsanwälte PartGmbB
(86) Internationale Anmeldenummer: PCT/EP2019/083657
(87) Internationale Veröffentlichungsnummer: WO 2020/126501

(56) Entgegenhaltungen:
- EP-A2- 2 090 273
- WO-A1-2019/091716
- US-A1- 2013 046 394

## Beschreibung

Die Erfindung betrifft ein elastisches Flächenmaterial mit einer Vorderseite und einer der Vorderseite gegenüberliegenden Rückseite und zumindest einem auf der Vorderseite angeordneten Funktionselement, das mit dem Flächenmaterial verbunden ist. Die Erfindung betrifft ebenfalls eine Bandage auf einem solchen Flächenmaterial sowie ein Verfahren zum Herstellen eines solchen Flächenmaterials. Ein solches Flächenmaterial wird insbesondere als Ausgangswerkstoff zum Herstellen von orthopädietechnischen Geräten oder Orthesen, insbesondere zum Herstellen von Manschetten oder Bandagen verwendet.

Manschetten, Bandagen oder Orthesen weisen in der Regel einen elastischen Grundkörper auf, der um die Gliedmaße oder einen Gelenkbereich herum angelegt werden kann. An dem Grundkörper kann ein Stabilisierungselement angeordnet sein, beispielsweise eine Metallschiene oder eine Kunststoffschiene. Bandagen oder Manschetten werden zur Unterstützung oder Stabilisierung der Gelenkfunktion oder der Muskulatur und Bändern angelegt. Dazu wird die Bandage über das zu schützende Gelenk oder die zu unterstützende Muskulatur gezogen oder darum gewickelt und fixiert. Bandagen lassen, im Gegensatz zu Immobilisationsorthesen, eine Bewegung der durch das Gelenk verbundenen Gliedmaßen relativ zueinander zu. Dazu weisen die Bandagen einen Grundkörper auf, der aus einem flexiblen Material besteht, beispielsweise einem Schaumstoff oder einem Textil. Zur Unterstützung der Stabilisationswirkung sind an dem Grundkörper Stabilisierungselemente angeordnet, die eine gegenüber dem Grundkörper erhöhte Festigkeit und Steifigkeit aufweisen, sodass neben einem Schutz gegen mechanische Einwirkung eine erhöhte Bewegungseinschränkung bewirkt wird.

Aus der US 2008/0039757 A1 ist eine flexible Bandage mit einem Rahmen mit einer Oberfläche zum Anlegen an einen Körperbereich bekannt, wobei der Rahmen eine Vielzahl an Öffnungen aufweist. An dem Rahmen selbst kann ein Gewebe oder ein Schaumstoff angebracht sein, beispielsweise durch Verschweißen, Verkleben oder durch Vernähen.

Die EP 876 130 B1 beschreibt eine orthopädische Stütze mit einer Halterung aus einem einteiligen Spritzgussteil mit Bereichen unterschiedlicher Dicke, um sich an den Umriss eines ausgewählten Teils eines Körpergliedes anzupassen. Eine Lage elastisches Material ist innerhalb der Halterung angebracht, um die Halterung zu polstern.

Die US 6,024,712 A beschreibt eine Sprunggelenkorthese mit einem inneren Textilteil, das sich zumindest teilweise um ein Sprunggelenk erstreckt. Eine Außenstütze ist mit dem Textil über ein Spritzgussverfahren verbunden und stellt eine zusätzliche Abstützung des Gelenkes gegen unbeabsichtigte Bewegungen dar.

Die CA 2,398,059 A1 beschreibt eine orthopädische Stütze mit einem flexiblen Innenteil und einer Außenstütze, die direkt auf das flexible Innenteil aufgeformt ist.

Die WO 2013/072064 A1 beschreibt eine orthopädietechnische Manschette zur Anlage an einem Körper mit einem flexiblen Grundkörper mit zumindest einem daran angeordneten Stabilisierungselement aus Kunststoff. Das Stabilisierungselement weist Bereiche mit einem gegenüber dem übrigen Querschnitt verringerten Querschnitt auf, in denen das Stabilisierungselement auf dem Grundkörper aufgeklebt und/oder stoffschlüssig mit dem Grundkörper verbunden ist. Das Stabilisierungselement kann auf einem flexiblen Trägermaterial aufgebracht sein, insbesondere aufgespritzt oder hinterspritzt sein. Das Trägermaterial bildet dann die Bereiche aus, die stoffschlüssig mit dem Grundkörper verbunden sind.

Bei der Verbindung von Polymeren mit elastischen, insbesondere textilen Flächenmaterialien wird häufig das Spritzgussverfahren angewendet, bei dem die Polymere tief in das textile Gewebe eindringen. Problematisch bei diesem Herstellverfahren ist die mangelnde Flexibilität, da für jede Ausgestaltung ein neues Werkzeug aufwendig hergestellt werden muss.

Die EP 2 090 273 A2 beschreibt eine Bandage aus einem Gestrick, die ein Stabilisierungselement für ein Gelenk aufweisen kann. Dieses kann beispielsweise auf der Außenseite der Bandage angeordnet und dann über eine Schnellverschlussvorrichtung fixiert werden. Daneben ist es auch möglich, dass das Stabilisierungselement in eine in der Bandage ausgebildeten Tasche angeordnet wird.

Die US 2013/0046394 A1 beschreibt einen Gel-Liner für eine Prothese. Auf der Linerinnenseite ist eine Mehrzahl von Elektroden zur Abnahme myoelektrischer Signale angeordnet. Auf der Außenseite des Liners ist ein Prothesenschaft angeordnet, dass den Liner umgibt

Aufgabe der vorliegenden Erfindung ist es daher, ein Flächenmaterial, eine Bandage sowie ein Verfahren zum Herstellen eines Flächenmaterials bereitzustellen, die eine dauerhafte und preisgünstige Verbindung eines Funktionselementes an dem Flächenmaterial bei einer hohen Individualisierungsrate ermöglichen.

Erfindungsgemäß wird diese Aufgabe durch ein Flächenmaterial mit den Merkmalen des Hauptanspruches, eine daraus hergestellte Bandage sowie ein Verfahren mit den Merkmalen des nebengeordneten Anspruchs gelöst. Vorteilhafte Ausgestaltungen und Weiterbildungen der Erfindung sind in den jeweiligen Unteransprüchen, der Beschreibung sowie den Figuren offenbart.

Das erfindungsgemäße elastische Flächenmaterial mit einer Vorderseite und einer der Vorderseite gegenüberliegenden Rückseite und zumindest einem auf der Vorderseite angeordneten Funktionselement, das mit dem Flächenmaterial verbunden ist, sieht vor, dass auf der Rückseite des Flächenmaterials dem Funktionselement gegenüberliegend und/oder auf der Vorderseite unterhalb des Funktionselementes und/oder innerhalb des Flächenmaterials ein Begrenzungselement zur Begrenzung der Elastizität des Flächenmaterials, insbesondere lokal in dem Bereich des Funktionselementes angeordnet ist. Die Rückseite eines elastischen Flächenmaterials wird insbesondere zur Auflage oder Anlage an einer Hautoberfläche eingesetzt, während ein Funktionselement in der Regel eine rigide oder abstehende Struktur aufweist, die besser an der Außenseite, also auf der dem Körper abgewandten Seite des Flächenmaterials, angeordnet ist. Die Anordnung eines Begrenzungselementes, das die Elastizität des Flächenmaterials begrenzt oder aufhebt, zumindest in der Ebene der Vorderseite und Rückseite hat den Vorteil, dass auch bei einem weniger tiefen Eindringen des Materials des Funktionselementes in die Struktur des elastischen Flächenmaterials keine Spannung und keine Relativbewegung beim Dehnen und wieder Zusammenziehen des Textils beim radialen oder longitudinalen Aufweiten des Flächenmaterials entsteht. Ein Verringern der Dicke des Flächenmaterials, also ein Aufeinanderzubewegen der Rückseite auf die Vorderseite, ist dabei unschädlich und muss durch das Begrenzungselement nicht verhindert werden. Eine Begrenzung der Elastizität vorwiegend in eine Richtung kann durch spezielle Ausgestaltungen des Begrenzungselementes erreicht werden, beispielsweise durch ein flexibles Gewebe, das in einer Richtung zugstarr und in der senkrecht dazu orientierten Richtung elastisch ist. Damit wird eine ausreichende Begrenzung insbesondere bei Funktionselementen erzielt, die sich überwiegend nur in eine Richtung erstrecken. Das Begrenzungselement verhindert oder vermindert ein Ausdehnen und Zusammenziehen des Flächenmaterials als Träger für das Funktionselement und verhindert oder reduziert somit eine Relativbewegung zwischen dem Funktionselement und dem Flächenmaterial, da keine oder verminderte Spannungen bei einer Dehnung des Flächenmaterials auf die Bindungsflächen oder den Anbindungsbereich des Funktionselementes mit dem Flächenmaterial übertragen werden. Dadurch wird verhindert, dass sich die physikalische Verbindung zwischen dem Funktionselement und dem Flächenmaterial lockert oder aufhebt, sodass die Dauerfestigkeit der Anbindung des Funktionselementes an dem Flächenmaterial beibehalten wird. Es hat sich überraschend gezeigt, dass selbst bei einer Versteifung nur auf einer Seite des Flächenmaterials, also auf der Vorderseite, innerhalb des Flächenmaterials oder auf der Rückseite, sich die Dauerfestigkeit der Verbindung des Funktionselementes mit dem Flächenmaterial wesentlich erhöht hat, nachdem das Flächenmaterial durch das Aufbringen oder Anordnen des Begrenzungselementes vorher partiell versteift wurde. Die partielle Rigidität des Flächenmaterials hat zudem den Vorteil, dass die Elastizität des Endproduktes in seiner Gesamtheit nur unwesentlich und nur in den Teilbereichen, in denen das Funktionselement angeordnet ist, eingeschränkt wird. Da die Funktionselemente in der Regel ebenfalls im Bereich der Anbindung an das elastische Flächenmaterial rigide ausgestaltet sind, ergibt sich keine merkliche Einschränkung in dem Tragekomfort des Flächenmaterials, wenn dieses zu einer orthopädietechnischen Einrichtung, beispielsweise einer Bandage oder einer Manschette, verarbeitet wird. Das Begrenzungselement kann sowohl auf der dem Funktionselement gegenüberliegenden Seite angeordnet sein als auch eine Zwischenschicht zwischen Funktionselement und Flächenmaterial bilden.

Das Flächenmaterial ist bevorzugt als Textil ausgebildet, beispielsweise ein Gestrick, Gelege oder Gewirk. Die Ausgestaltung als Abstandsgewirk ist ebenfalls vorgesehen. Alternativ kann das Flächenmaterial als Schaumstoff ausgebildet sein oder Schaumstoffanteile enthalten. Als Flächenmaterial wird ein Material angesehen, das eine überwiegend zweidimensionale Ausdehnung aufweist, also eine große Fläche bei einer geringen Materialstärke aufweist.

Das Begrenzungselement weist in einer Weiterbildung der Erfindung eine Außenkontur auf, die der Außenkontur des Funktionselementes entspricht oder die Außenkontur des Funktionselementes zumindest teilweise, bevorzugt vollständig einschließt. Entspricht die Außenkontur des Begrenzungselementes der Außenkontur des Funktionselementes, wird nur ein minimaler Bereich des Flächenmaterials versteift, wodurch das übrige Elastizitätsverhalten des Flächenmaterials nicht beeinträchtigt wird. Bei einem vergrößerten Flächenbereich des Begrenzungselementes im Vergleich zur Außenkontur des Funktionselementes wird eine größere Fläche des Flächenmaterials versteift, was einerseits die Positionierung des Funktionselementes erleichtert und andererseits Spannungen in dem Randbereich des Flächenmaterials am Übergang zu dem Funktionselement verhindert und dadurch eine erhöhte Dauerhaltbarkeit bereitstellt. Alternativ kann die Außenkontur des Begrenzungselementes auch nur die Bereiche des Funktionselemtens abdecken, die rigide ausgebildet sind. Andere Bereiche des Funktionselementes wie etwa Federn, die selbst eine ausreichende Elastizität ausbilden, können der Bewegung des elastischen Flächenmaterials folgen und lösen sich nicht so schnell ab, sodass hier eine Begrenzung der Elastizität des Flächenmaterials nicht zwingend erforderlich ist.

Das Begrenzungselement ist bevorzugt stoffschlüssig mit dem Flächenmaterial verbunden, beispielsweise verschweißt oder verklebt.

Das Begrenzungselement kann als Folie auf das Flächenmaterial aufgeklebt oder aufgeschweißt, bevorzugt vollflächig aufgeklebt oder verschweißt werden. Ebenfalls ist es möglich, dass das Begrenzungselement durch eine Klebstoffschicht oder durch eine Vielzahl von Klebstoffstreifen oder Klebstoffpunkten oder ein Klebstoffgitter ausgebildet wird, das auf dem Flächenmaterial aufgebracht wird, so dass es an der Oberfläche haftet und/oder in das Flächenmaterial eindringt. Alternativ kann das Begrenzungselement als ein unelastisches, aber flexibles Textil, ein Durchdringungselement, das das Flächenmaterial durchdringt und einen Vorsprung auf der gegenüberliegenden, insbesondere der Vorderseite ausbildet, als ein leitfähiges Textil oder Band oder Kabel, beispielsweise eine metallische Fadenkomponente aufweisend, ausgebildet sein, um zwar eine ausreichende Flexibilität zu ermöglichen, die Elastizität jedoch in dem Teilbereich, in dem das Begrenzungselement aufgebracht ist, zumindest zu vermindern oder aufzuheben. Das Begrenzungselement kann auch als Sensor, beispielsweise kapazitiver Sensor ausgebildet sein, um über die Begrenzungsfunktion bezüglich der Elastizität hinaus einen funktionalen Beitrag zur Ausgestaltung einer orthopädietechnischen Komponente zu liefern.

Eine Weiterbildung der Erfindung sieht vor, dass mehrere Begrenzungselemente getrennt voneinander auf dem Flächenmaterial angeordnet sind, um die Elastizität und Flexibilität des Gesamtflächenmaterials nicht oder nur möglichst wenig einzuschränken. Die Begrenzungselemente können beispielsweise in einer Längserstreckung hintereinander, getrennt voneinander auf dem Flächenmaterial aufgebracht und daran festgelegt sein. Ebenfalls ist es möglich, bei einer hülsenartigen Ausgestaltung des fertigen Produktes über den Umfang verteilt, in Längsrichtung orientierte Begrenzungselemente vorzusehen, die ein radiales Aufweiten in den Zwischenbereichen zwischen den Begrenzungselementen ermöglichen, eine Längung in Längserstreckung jedoch zumindest bereichsweise verhindern.

Die Begrenzungselemente oder das Begrenzungselement sind bevorzugt flexibel ausgebildet, um das Flächenmaterial an unterschiedliche Oberflächen, insbesondere Körperoberflächen, anpassen zu können. Das heißt, dass das Begrenzungselement biegsam ist, jedoch keine oder nur eine verminderte Längenveränderung in der Ebene zulässt.

Eine Weiterbildung der Erfindung sieht vor, dass das Funktionselement auf dem Flächentextil aufgedruckt oder mittels eines additiven Herstellverfahrens auf dem Flächenmaterial aufgebracht ist, also in einem schichtweisen Auftragsprozess oder additiven Herstellverfahren auf dem Flächentextil aufgebracht und dort formschlüssig und/oder stoffschlüssig verbunden wird. Das Funktionselement kann beispielsweise als Elastomer oder aus einem Elastomer ausgebildet und insbesondere in einem additiven Herstellverfahren auf dem Textil aufgebracht werden. Über additive Herstellverfahren, z.B. den sogenannten 3D-Druck, beispielsweise mit einem SLA-Drucker oder FDM-Drucker, ist es möglich, individuelle Produkte herzustellen, indem die Funktionselemente an den jeweiligen Nutzer angepasst auf das Flächenmaterial aufgebracht, z.B. aufgedruckt werden und dieses anschließend zu einem Endprodukt verarbeitet wird. Auch ist es möglich, das Flächenmaterial zunächst in eine Grundform zu bringen, beispielsweise als Kniemanschette, Ellenbogenmanschette oder Wadenmanschette und anschließend auf die bereits vorgeformte Grundform das jeweils gewünschte Funktionselement aufzubringen oder aufzudrucken. Das Aufbringen oder Aufdrucken kann beispielsweise nach dem Anpassen der Bandage an den jeweiligen Körper erfolgen, sodass die Positionierung der Funktionselemente auf dem Flächentextil hochgenau und individuell erfolgen kann.

Das Funktionselement kann als Feder, Schiene, Versteifungselement, Bauteilaufnahme, Kanal, Haken, Sensorhalter, Kabelführung, Kabel, Leiter, Klipselement, Schnalle, Reißverschluss, Protektor, Polster, Luftpolster, Elastomerelement und/oder Sensor ausgebildet sein.

Die Erfindung betrifft ebenfalls eine Bandage aus einem Flächenmaterial wie es oben beschrieben worden ist. Eine Weiterbildung der Bandage sieht vor, dass diese hülsenartig mit einem geschlossenen Querschnitt oder aus einem flächigen Grundkörper mit Verbindungseinrichtungen ausgebildet ist, die an einander gegenüberliegenden Endbereichen angeordnet sind. Sofern die Bandage einen geschlossenen Querschnitt aufweist, kann dieser unterbrochen ausgebildet sein, beispielsweise um Ausnehmungen für Vorsprünge zu bilden. Bei einer Ausgestaltung der Bandage als Kniebandage kann eine Ausnehmung im Bereich der Patella und/oder Kniekehle ausgebildet sein, bei einer Ellenbogenbandage kann eine Ausnehmung beispielsweise im Bereich der Ellenbeuge angeordnet sein. Mit einem geschlossenen Querschnitt kann die Bandage über ein Körperglied gezogen werden. Alternativ ist es möglich, die Bandage mit einem offenen Querschnitt auszubilden und nach dem Anlegen an den Körper zu schließen und in der jeweiligen gewünschten Position und in der gewünschten Spannung zu fixieren. Dazu sind an einander gegenüberliegenden Kanten oder Endbereichen des Flächenmaterials Verbindungseinrichtungen wie Klettverschlüsse, Schlaufen oder Schnallen angeordnet, um den zunächst offenen Querschnitt des Zuschnitts zu schließen.

Das Verfahren zum Herstellen eines oben beschriebenen Flächenmaterials sieht vor, dass das Funktionselement mit dem Flächenmaterial verbunden wird und zumindest ein Begrenzungselement zwischen dem Funktionselement und dem Flächenmaterial und/oder innerhalb des Flächenelementes insbesondere im Bereich des Funktionselementes und/oder auf der dem Funktionselement gegenüberliegenden Seite des Flächenmaterials angeordnet und verbunden wird und durch das Begrenzungselement die Elastizität des Flächenmaterials zumindest verringert wird. Das Verbinden des Begrenzungselementes mit dem Flächenmaterial erfolgt dabei bevorzugt vor dem Verbinden des Funktionselementes mit dem Flächenmaterial.

Das Funktionselement kann in einem additiven Herstellverfahren, z.B. 3D-Druckverfahren, auf das Flächenmaterial oder das Begrenzungselement aufgebracht oder aufgedruckt werden. Bevorzugt wird das Begrenzungselement vollflächig mit dem Flächenmaterial verbunden, beispielsweise vollflächig verschweißt oder verklebt, um eine im Anbindungsbereich des Funktionselementes auftretende Lockerung und Relativbewegung des Flächenmaterials zu verhindern.

Bevorzugt wird das Funktionselement innerhalb der Außenkontur des Begrenzungselementes angeordnet, um an den Übergangsstellen des Funktionselementes zu dem Flächenelement keine Relativbewegungen zwischen dem Flächenmaterial und dem Funktionselement zu ermöglichen und dadurch die Dauerhaltbarkeit des befestigten Funktionselementes zu erhöhen.

Das Begrenzungselement wird bevorzugt stoffschlüssig mit dem Flächenmaterial verbunden, um eine dauerhafte Elastizitätsbegrenzung zu erreichen.

Nachfolgend wird die Erfindung anhand der Figuren näher erläutert. Es zeigen:
- Figur 1: eine Schnittdarstellung einer Bandage;
- Figur 2: eine Seitenansicht einer Kniebandage;
- Figur 3: eine Schnittdarstellung und eine Draufsicht auf ein Flächenmaterial;
- Figur 4: eine Variante der Figur 3;
- Figur 5: eine Variante der Figur 3;
- Figur 6: eine Variante der Figur 2;
- Figur 7: eine Variante der Figur 5; sowie
- Figur 8: eine Schnittdarstellung durch ein Flächenmaterial

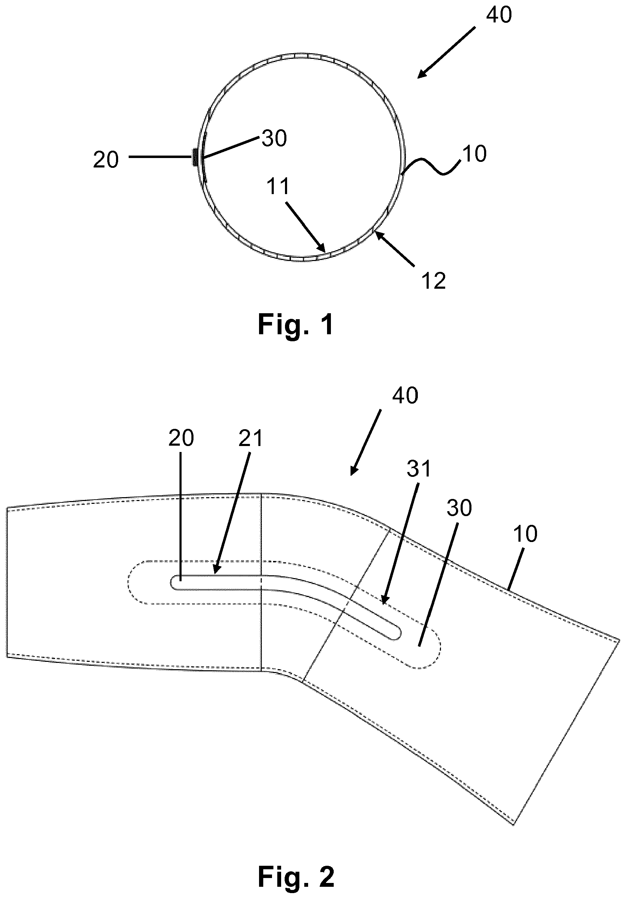

In der Figur 1 ist ein Querschnitt durch eine hülsenförmige Bandage 40 gezeigt, die einen Grundkörper aus einem Flächenmaterial 10 aufweist. Das Flächenmaterial 10 ist aus einem elastischen Werkstoff, insbesondere einem elastischen Textil oder einem Schaumstoff hergestellt und weist in dem dargestellten Ausführungsbeispiel einen geschlossenen Querschnitt auf, so dass die Bandage 40 insgesamt eine hülsenförmige Form aufweist. Im Verlauf der Längserstreckung der Bandage 40 können sich der Querschnitt und die Form des Grundkörpers verändern. Das elastische Flächenmaterial 10 weist eine dem Nutzer oder Patienten zugewandte Innenseite, die als Rückseite 11 bezeichnet wird, und eine im angelegten Zustand der Bandage 40 dem Patienten abgewandte Außenseite oder Vorderseite 12 auf, die der Rückseite 11 gegenüberliegt. Auf der Vorderseite 12 des Grundkörpers ist ein Funktionselement 20 angeordnet, das mit dem Flächenmaterial 10 des Grundkörpers verbunden ist. Auf der Rückseite 11 dem Funktionselement 20 gegenüberliegend ist ein Begrenzungselement 30 angeordnet, das zur Begrenzung der Elastizität des Flächenmaterials 10 dient. Durch das Begrenzungselement 30 wird die Dehnbarkeit des Flächenmaterials 10 im Bereich des Funktionselementes 20 lokal verringert, gegebenenfalls auf null verringert, so dass bei einer Beanspruchung des elastischen Flächenmaterials 10 durch eine Bewegung oder durch das Anlegen der Bandage 40 an den Patienten keine oder nahezu keine Dehnung in dem Bereich des Funktionselementes 20 auftritt, die Dehnbarkeit in den übrigen Bereichen der Bandage 40 jedoch nicht beeinträchtigt wird. Dadurch wird das Aufbringen von Scherspannungen im Bereich der Verbindung zwischen dem Funktionselement 20 und dem Flächenmaterial 10 verhindert oder zumindest signifikant vermindert, so dass das Funktionselement 20 sicher und dauerhaft an der Außenseite oder Vorderseite12 des Flächenmaterials 10 befestigt werden kann.

Figur 2 zeigt eine Ausgestaltung und Verwendung des elastischen Flächenmaterials 10 als eine Bandage 40, die ein Kniegelenk überspannen soll. Die Bandage 40 ist hülsenförmig aufgebaut und weist eine Vorflexion auf, um eine übermäßige Dehnung im Kniescheibenbereich, also auf der Vorderseite des Knies, und eine übermäßige Stauchung im Kniekehlenbereich, also auf der Rückseite des Knies, zu verhindern. Der Unterschenkelbereich, der in der Figur 2 links dargestellt ist, weist einen sich zum distalen Ende hin verjüngenden Querschnitt auf, der Oberschenkelbereich erweitert sich zum proximalen Ende hin. Auf der Außenseite ist, das Kniegelenk überspannend, ein Funktionselement 20 in Gestalt einer Feder, Schiene oder eines Versteifungselementes aufgebracht, insbesondere aufgeschweißt oder aufgeklebt. Das Funktionselement 20 kann auch als Kanal oder Bauteilaufnahme zur Aufnahme einer Schiene, einer Feder oder einer Gelenkschiene ausgebildet sein, ebenfalls kann es als Haken, Halter für Sensoren, Kabel, als Kabel selbst, als Leiter, als Klipselement, Schnalle, Reißverschluss, Protektor oder als Sensor ausgebildet sein. Im dargestellten Ausführungsbeispiel mit dem Funktionselement 20 als Schiene oder Versteifungselement wird eine verbesserte Führung des Gelenkes im angelegten Zustand erreicht. Auf der Innenseite oder Rückseite 11 des Flächenmaterials ist das Begrenzungselement 30 angeordnet, das mit einer durchbrochenen Linie dargestellt ist. Das Begrenzungselement 30 ist bevorzugt flexibel, jedoch nicht elastisch und kann als Folie, Klebstoffschicht und elastisches Textil oder Ähnliches ausgebildet sein. Es besteht auch die Möglichkeit, dass mehrere Begrenzungselemente 30 übereinander angeordnet sind, um die Begrenzungswirkung zu verstärken. Das Begrenzungselement 30 kann stoffschlüssig mit dem Flächenmaterial verbunden sein, beispielsweise aufgeklebt oder aufgeschweißt oder aber innerhalb des Flächenmaterials 10 integriert sein, beispielsweise eingegossen, eingeschäumt oder einlaminiert. Das Funktionselement 20 weist eine Außenkontur 21 auf, die innerhalb einer Außenkontur 31 des Begrenzungselementes 30 liegt, so dass kein elastisches Flächenmaterial 10 in dem Bereich, in dem das Funktionselement 20 auf der Außenseite oder Vorderseite 12 des Flächenmaterials 10 angebracht ist, eine elastische oder eine verstärkt elastische Dehnung ausübt. In dem dargestellten Ausführungsbeispiel steht die Außenkontur 31 des Begrenzungselementes 30 allseitig über die Außenkontur 21 des Funktionselementes 20 über, um sicherzustellen, dass an keiner Stelle eine ungewollte Scherspannung auftritt.

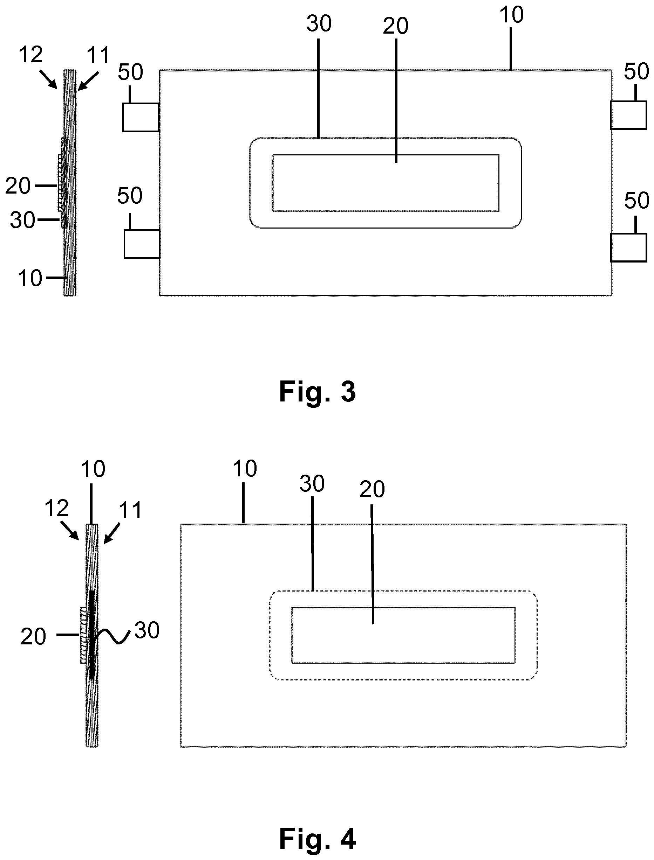

Figur 3 zeigt ein elastisches Flächenmaterial 10 als Grundmaterial. An zwei einander gegenüberliegenden Seitenkanten des Flächenmaterials 10 sind Verbindungseinrichtungen 50 in Gestalt von Klettverschlussbereichen, Druckknöpfen, Schnallen, Schlaufen oder dergleichen angeordnet oder ausgebildet, um die Endbereiche miteinander zu verbinden und einen geschlossenen Querschnitt auszubilden, um beispielsweise das Flächenmaterial 10 zu einer Bandage 40 mit einem geschlossenen Querschnitt auszugestalten, die leicht angelegt und wieder abgelegt und hinsichtlich ihres Durchmessers oder Umfanges anpassbar ist. In der linken Schnittdarstellung ist zu erkennen, dass das Funktionselement 20 unmittelbar auf dem Begrenzungselement 30 aufgebracht ist, beispielsweise aufgeklebt, aufgedruckt oder aufgeschweißt, wobei das Begrenzungselement 30 auf der Vorderseite 12 des Flächenmaterials 10 befestigt ist. Das Begrenzungselement 30 ist teilweise in das Material des Flächenmaterials 10 eingebettet und steht leicht über die Vorderseite 12 hinaus. Alternativ kann das Begrenzungselement 30 vollständig in dem Flächenmaterial 10 integriert sein und bündig mit der Vorderseite 12 abschließen, so dass eine gemeinsame Ebene zwischen der Vorderseite 12 und der Außenseite des Begrenzungselementes 30 gebildet wird, auf der dann das Funktionselement 20 aufgebracht, insbesondere aufgeklebt oder aufgeschweißt wird. In der rechten Darstellung der Figur 3 ist zu erkennen, dass der Außenumfang des Begrenzungselementes 30 größer als der des Funktionselementes 20 ist und die Projektion des Funktionselementes 20 innerhalb der Projektion des Begrenzungselementes 30 liegt.

In der Figur 4 ist eine Variante des Flächenmaterials 10 gezeigt. In der Schnittdarstellung ist zu erkennen, dass das Begrenzungselement 30 innerhalb des Flächenmaterials eingebettet und das Funktionselement 20 auf dem Flächenmaterial 10 auf der Vorderseite 12 angeordnet und befestigt ist. Durch die Anordnung des Begrenzungselementes 30 innerhalb des Flächenmaterials 10 hinter dem Funktionselement 20 wird beim Aufbringen einer Zugkraft über das Flächenmaterial 10 eine Weiterleitung von Scherkräften an der Außenseite oder Vorderseite 12 verhindert und eine Scherspannung im Bereich der Befestigung des Funktionselementes 20 verhindert oder zumindest vermindert. Das Begrenzungselement 30 ist hier ebenfalls großflächiger als das Funktionselement 20, die Außenkontur 21 des Funktionselementes 20 liegt innerhalb der Außenkontur 31 des Begrenzungselementes 30.

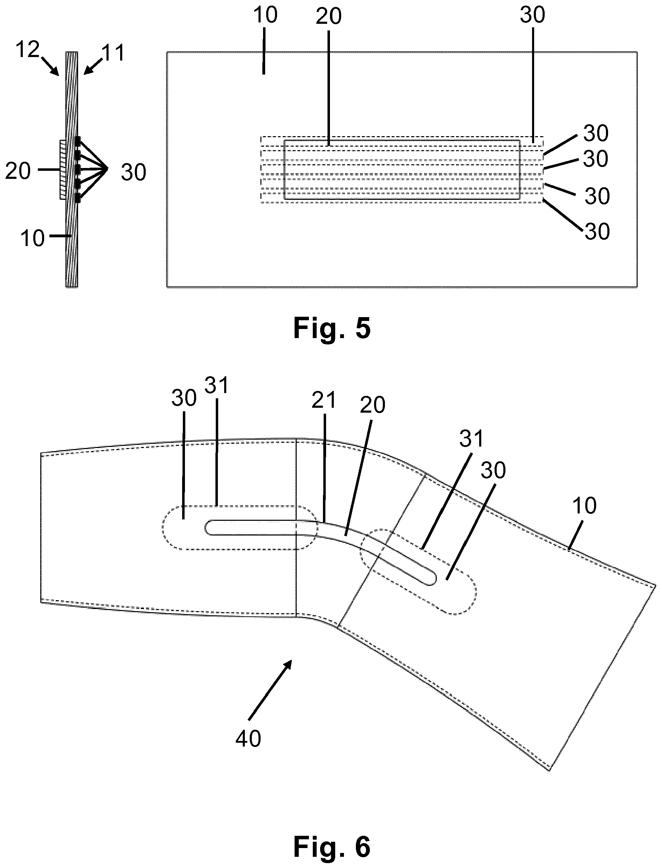

Figur 5 zeigt eine weitere Variante, bei der statt eines einzelnen Begrenzungselementes 30 auf der Rückseite 11 eine Vielzahl, hier fünf Begrenzungselemente 30 angeordnet sind, die streifenförmig positioniert und mit dem Flächenmaterial 10 verbunden sind. Eine solche Anordnung ist insbesondere dann sinnvoll, wenn Zugkräfte nur in Längserstreckung der Begrenzungselemente 30 und des Funktionselementes 20 zu erwarten sind und/oder sich das Begrenzungselement 30 überwiegend in Längserstreckung erstreckt. Eine gitterförmige Anordnung von streifenförmigen Begrenzungselementen 30 ist ebenfalls möglich. Begrenzungselemente 30 sind auf der Rückseite 11 des Flächenmaterials 10 in der Art und Weise befestigt, wie sie im Zusammenhang mit dem Begrenzungselement 30 der Figur 3 beschrieben wurden. In der, wie dargestellt, leicht nach Innen überstehenden Anordnung der Begrenzungselemente 30 können diese auch bündig mit der Rückseite abschließend ausgebildet sein.

Figur 6 zeigt eine Variante der Figur 2 mit zwei Begrenzungselementen 30, die an den distalen und proximalen Endbereichen des Funktionselementes 20 auf der Rückseite 11 oder innerhalb des Flächenmaterials 10 angeordnet sind, wie durch die unterbrochenen Linien angedeutet ist. Im Bereich der natürlichen Gelenkachse, hier Kniegelenksachse, sind keine Begrenzungselemente 30 angeordnet, um eine freie Beweglichkeit in diesem Bereich zuzulassen. Es besteht die Möglichkeit, dass das Funktionselement 30, beispielsweise eine Feder oder eine in diesem Bereich elastische Schiene oder auch eine Gelenkschiene, in dem Bereich, der nicht von den Begrenzungselementen 30 abgedeckt ist, nicht mit der Vorderseite 12 oder Außenseite des Flächenmaterials 10 verbunden ist, um so eine Relativbewegung zwischen dem Funktionselement 20 und dem Grundkörper 10 der Bandage 40 in diesem Bereich zu ermöglichen. Das Funktionselement 20 ragt in diesem Ausführungsbeispiel bereichsweise über die Außenkontur 31 des Begrenzungselementes hinaus.

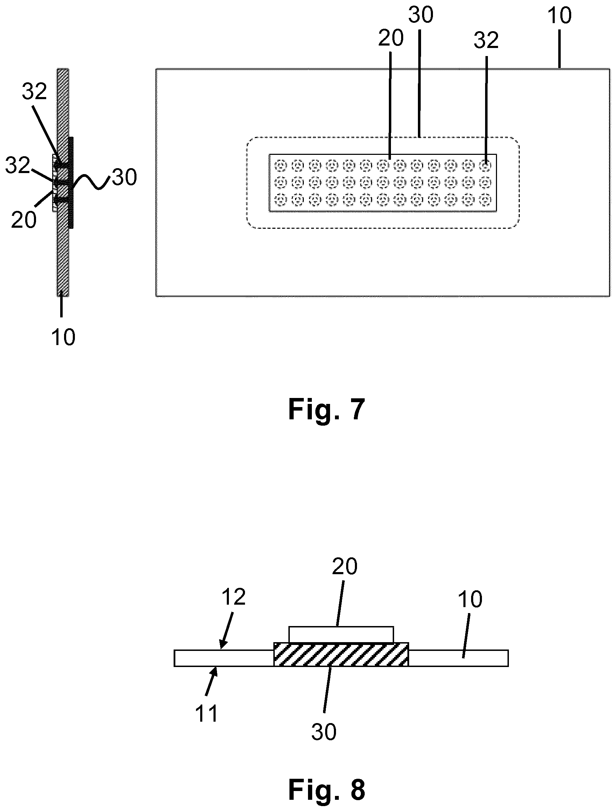

Eine weitere Variante der Erfindung ist in der Figur 7 gezeigt, bei der auf der Rückseite 11 des Flächenmaterials 10 das Begrenzungselement 30 angeordnet ist. Das Begrenzungselement 30 weist Durchdringungselemente 32 in Gestalt von Stiften auf, die das Flächenmaterial 10 durchdringen und über die Vorderseite 12 hinausragen. Im Bereich der Durchdringung können die Durchdringungselemente 32 Vorsprünge, Befestigungselemente, Aufnahmen für Klipse oder ähnliche Formschlusselemente oder einfach nur tellerartige oder pilzartige Verbreiterungen aufweisen, um zusätzlich zu einer stoffschlüssigen Befestigung des Begrenzungselementes 30 auf der Rückseite 11 eine formschlüssige Befestigung über die Durchdringungselemente 32 zu erreichen. Grundsätzlich ist es auch möglich, auf eine stoffschlüssige Verbindung des Begrenzungselementes 30 mit dem Flächenmaterial 10 zu verzichten. Die Durchdringungselemente 32 dienen zur Befestigung des Funktionselementes 20 oder zur Unterstützung der Befestigung an dem Flächenmaterial 10. Die Befestigung kann stoffschlüssig auf der Vorderseite 12 des Flächenmaterials 10 allein oder in Verbindung mit einer formschlüssigen oder auch stoffschlüssigen Verbindung mit den Durchdringungselementen 32 erfolgen.

Figur 8 zeigt eine weitere Variante der Erfindung, bei der das Begrenzungselement 30 innerhalb des Flächenmaterials 10 integriert ist und dieses im Bereich des Funktionselementes 20 vollständig durchdringt. Das Begrenzungselement 30 kann als zunächst flüssiger Klebstoff in einem definierten Bereich auf einen textilen Grundkörper aufgebracht werden und das Textil durchdringen. Das Funktionselement 20, z.B. ein Versteifungselement, kann über den Klebstoff des Begrenzungselementes 30 auf dem Flächenmaterial 10 fixiert werden. Der Klebstoff versteift das Textil und verhindert eine Bewegung in dem Bereich, in dem der Klebstoff aushärtet. Statt das Funktionselement 20 über den Klebstoff zu fixieren, kann es auch auf dem Begrenzungselement aufgedruckt oder über ein additives Herstellverfahren aufgebracht werden. Das Begrenzungselement 30 kann auf der Vorderseite 12 bündig mit dem Flächenmaterial 10 abschließen oder darüber hinausstehen, wie dies in der Figur 8 gezeigt ist. Auf der Innenseite 11 schließt das Begrenzungselement 30 vorzugsweise bündig mit dem Flächenmaterial 10 ab.

Bevorzugt ist das Begrenzungselement 30 ähnlich flexibel wie das Flächenmaterial 10, weist jedoch eine wesentlich verringerte Dehnbarkeit auf.

## Patentansprüche

1. Elastisches Flächenmaterial (10) mit einer Vorderseite (12) und einer der Vorderseite (12) gegenüberliegenden Rückseite (11) und zumindest einem auf der Vorderseite (12) angeordneten Funktionselement (20), das mit dem Flächenmaterial (10) verbunden ist, **dadurch gekennzeichnet, dass** auf der Rückseite (11) des Flächenmaterials (10) dem Funktionselement (20) gegenüberliegend und/oder auf der Vorderseite (12) unterhalb des Funktionselementes (20) oder innerhalb des Flächenmaterials (10) unterhalb des Funktionselementes (20) ein Begrenzungselement (30) zur Begrenzung der Elastizität des Flächenmaterials (10) angeordnet ist.

2. Flächenmaterial nach Anspruch 1, **dadurch gekennzeichnet, dass** das Begrenzungselement (30) eine Außenkontur (31) aufweist, die der Außenkontur (21) des Funktionselementes (20) entspricht oder die Außenkontur (21) des Funktionselementes (20) einschließt.

3. Flächenmaterial nach Anspruch 1 oder 2, **dadurch gekennzeichnet, dass** das Begrenzungselement (30) stoffschlüssig mit dem Flächenmaterial (10) verbunden ist.

4. Flächenmaterial nach einem der voranstehenden Ansprüche, **dadurch gekennzeichnet, dass** es als ein Textil ausgebildet ist.

5. Flächenmaterial nach einem der voranstehenden Ansprüche, **dadurch gekennzeichnet, dass** das Begrenzungselement (30) als Folie, Klebstoffschicht, unelastisches Textil, Durchdringungselement (32) mit Vorsprung, leitfähiges Textil, Band, Kabel oder Sensor ausgebildet ist.

6. Flächenmaterial nach einem der voranstehenden Ansprüche, **dadurch gekennzeichnet, dass** mehrere Begrenzungselemente (30) getrennt voneinander auf dem Flächenmaterial (10) angeordnet sind.

7. Flächenmaterial nach einem der voranstehenden Ansprüche, **dadurch gekennzeichnet, dass** das Begrenzungselement (30) flexibel ausgebildet ist.

8. Flächenmaterial nach einem der voranstehenden Ansprüche, **dadurch gekennzeichnet, dass** das Funktionselement (20) auf dem Flächenmaterial (10) mit einem additiven Herstellverfahren insbesondere als Elastomerelement aufgebracht ist.

9. Flächenmaterial nach einem der voranstehenden Ansprüche, **dadurch gekennzeichnet, dass** das Funktionselement (20) als Feder, Schiene, Versteifungselement, Bauteilaufnahme, Kanal, Haken, Sensorhalter, Kabelführung, Kabel, Leiter, Klipselement, Schnalle, Reißverschluss, Protektor, Sensor, Polster oder Luftpolster ausgebildet ist.

10. Bandage aus einem Flächenmaterial (10) nach einem der voranstehenden Ansprüche.

11. Bandage nach Anspruch 10, **dadurch gekennzeichnet, dass** sie hülsenartig mit einem geschlossenen Querschnitt oder aus einem flächigen Grundkörper mit an einander gegenüberliegenden Endbereichen angeordneten Verbindungseinrichtungen (50) ausgebildet ist.

12. Verfahren zum Herstellen eines Flächenmaterials (10) nach einem der Ansprüche 1 bis 8, **dadurch gekennzeichnet, dass** das Funktionselement (20) mit dem Flächenmaterial (10) verbunden wird und zumindest ein Begrenzungselement (30) zwischen dem Funktionselement (20) und dem Flächenmaterial (10) und/oder auf der dem Funktionselement (20) gegenüberliegenden Seite des Flächenmaterials (10) oder in das Flächenmaterials (10) eingebracht und mit dem Flächenmaterial (10) verbunden wird und dass durch das Begrenzungselement (30) die Elastizität des Flächenmaterials (10) zumindest verringert wird.

13. Verfahren nach Anspruch 12, **dadurch gekennzeichnet, dass** das Funktionselement (20) in einem additiven Herstellverfahren auf das Flächenmaterial (10) oder das Begrenzungselement (30) aufgebracht wird.

14. Verfahren nach Anspruch 12 oder 13, **dadurch gekennzeichnet, dass** das Begrenzungselement (30) vollflächig mit dem Flächenmaterial (10) verbunden wird.

15. Verfahren nach einem der Ansprüche 12 bis 14, **dadurch gekennzeichnet, dass** das Funktionselement (20) innerhalb der Außenkontur (31) des Begrenzungselementes (30) angeordnet wird.

16. Verfahren nach einem der Ansprüche 12 bis 15, **dadurch gekennzeichnet, dass** das Begrenzungselement (30) stoffschlüssig mit dem Flächenmaterial (10) verbunden wird.

## Claims

1. An elastic sheet material (10) with a front side (12), a rear side (11) situated opposite the front side (12), and at least one functional element (20) arranged on the front side (12) and connected to the sheet material (10), **characterized in that** a limiting element (30) for limiting the elasticity of the sheet material (10) is arranged on the rear side (11) of the sheet material (10) opposite the functional element (20) and/or on the front side (12) below the functional element (20) or inside the sheet material (10) below the functional element (20).

2. The sheet material as claimed in claim 1, **characterized in that** the limiting element (30) has an outer contour (31) which corresponds to the outer contour (21) of the functional element (20) or encloses the outer contour (21) of the functional element (20).

3. The sheet material as claimed in claim 1 or 2, **characterized in that** the limiting element (30) is materially bonded to the sheet material (10).

4. The sheet material as claimed in one of the preceding claims, **characterized in that** it is in the form of a textile.

5. The sheet material as claimed in one of the preceding claims, **characterized in that** the limiting element (30) is in the form of a film, adhesive layer, inelastic textile, penetrating element (32) with projection, conductive textile, band, cable or sensor.

6. The sheet material as claimed in one of the preceding claims, **characterized in that** a plurality of limiting elements (30) are arranged separate from one another on the sheet material (10).

7. The sheet material as claimed in one of the preceding claims, **characterized in that** the limiting element (30) has a flexible form.

8. The sheet material as claimed in one of the preceding claims, **characterized in that** the functional element (20) is applied, in particular in the form of an elastomer element, to the sheet material (10) by an additive production process.

9. The sheet material as claimed in one of the preceding claims, **characterized in that** the functional element (20) is in the form of a spring, rail, stiffening element, component receptacle, duct, hook, sensor mount, cable guide, cable, conductor, clip element, clasp, zip fastener, protector, sensor, cushion and/or air cushion.

10. A bandage made of a sheet material (10) as claimed in one of the preceding claims.

11. The bandage as claimed in claim 10, **characterized in that** it has a sheath-like form with a closed cross section or is formed from a sheet-like basic body with connecting devices (50) arranged at opposite end regions.

12. A method for producing a sheet material (10) as claimed in one of claims 1 to 8, **characterized in that** the functional element (20) is connected to the sheet material (10) and at least one limiting element (30) is introduced between the functional element (20) and the sheet material (10) and/or on that side of the sheet material (10) which is situated opposite the functional element (20), and is connected to the sheet material (10), and **in that** the limiting element (30) at least lessens the elasticity of the sheet material (10).

13. The method as claimed in claim 12, **characterized in that** the functional element (20) is applied to the sheet material (10) or the limiting element (30) in an additive production process.

14. The method as claimed in claim 12 or 13, **characterized in that** the limiting element (30) is connected to the sheet material (10) over its entire surface area.

15. The method as claimed in one of claims 12 to 14, **characterized in that** the functional element (20) is arranged inside the outer contour (31) of the limiting element (30).

16. The method as claimed in one of claims 12 to 15, **characterized in that** the limiting element (30) is materially bonded to the sheet material (10).

## Revendications

1. Matériau en feuille élastique (10) présentant une face avant (12) et une face arrière (11), opposée à la face avant (12), et au moins un élément fonctionnel (20) disposé sur la face avant (12) et relié au matériau en feuille (10),
**caractérisé en ce qu'**un élément de limitation (30) destiné à limiter l'élasticité du matériau en feuille (10) est disposé sur la face arrière (11) du matériau en feuille (10) à l'opposé de l'élément fonctionnel (20) et/ou sur la face avant (12) en dessous de l'élément fonctionnel (20) ou à l'intérieur du matériau en feuille (10) en dessous de l'élément fonctionnel (20).

2. Matériau en feuille selon la revendication 1,
**caractérisé en ce que** l'élément de limitation (30) présente un contour extérieur (31) qui correspond au contour extérieur (21) de l'élément fonctionnel (20) ou qui englobe le contour extérieur (21) de l'élément fonctionnel (20).

3. Matériau en feuille selon la revendication 1 ou 2,
**caractérisé en ce que** l'élément de limitation (30) est relié au matériau en feuille (10) par une liaison par coopération de matière.

4. Matériau en feuille selon l'une des revendications précédentes, **caractérisé en ce qu'**il est conçu comme un textile.

5. Matériau en feuille selon l'une des revendications précédentes, **caractérisé en ce que** l'élément de limitation (30) est conçu comme un film, une couche de colle, un textile inélastique, un élément de pénétration (32) avec une saillie, un textile conducteur, une bande, un câble ou un capteur.

6. Matériau en feuille selon l'une des revendications précédentes, **caractérisé en ce que** plusieurs éléments de limitation (30) sont disposés séparément les uns des autres sur le matériau en feuille (10).

7. Matériau en feuille selon l'une des revendications précédentes, **caractérisé en ce que** l'élément de limitation (30) est flexible.

8. Matériau en feuille selon l'une des revendications précédentes, **caractérisé en ce que** l'élément fonctionnel (20) est appliqué sur le matériau en feuille (10), en particulier sous forme d'élément élastomère, par un procédé de fabrication additive.

9. Matériau en feuille selon l'une des revendications précédentes, **caractérisé en ce que** l'élément fonctionnel (20) est conçu comme un ressort, un rail, un élément de rigidification, un logement de composant, un canal, un crochet, un support de capteur, un guide-câble, un câble, un conducteur, un élément à clipser, une boucle, une fermeture à glissière, un protecteur, un capteur, un coussin ou un coussin d'air.

10. Bandage en matériau en feuille (10) selon l'une des revendications précédentes.

11. Bandage selon la revendication 10,
**caractérisé en ce qu'**il est réalisé à la manière d'un manchon avec une section transversale fermée ou à partir d'un corps de base en feuille avec des moyens de liaison (50) disposés sur des zones d'extrémité opposées.

12. Procédé de fabrication d'un matériau en feuille (10) selon l'une des revendications 1 à 8,
**caractérisé en ce que** l'élément fonctionnel (20) est relié au matériau en feuille (10), et au moins un élément de limitation (30) est inséré entre l'élément fonctionnel (20) et le matériau en feuille (10) et/ou est appliqué sur le côté du matériau en feuille (10) opposé à l'élément fonctionnel (20) ou est introduit dans le matériau en feuille (10) et est relié au matériau en feuille (10), et
**en ce que** l'élément de limitation (30) réduit au moins l'élasticité du matériau en feuille (10).

13. Procédé selon la revendication 12,
**caractérisé en ce que** l'élément fonctionnel (20) est appliqué sur le matériau en feuille (10) ou sur l'élément de limitation (30) par un procédé de fabrication additive.

14. Procédé selon la revendication 12 ou 13,
**caractérisé en ce que** l'élément de limitation (30) est relié sur toute sa surface au matériau en feuille (10).

15. Procédé selon l'une des revendications 12 à 14,
**caractérisé en ce que** l'élément fonctionnel (20) est disposé à l'intérieur du contour extérieur (31) de l'élément de limitation (30).

16. Procédé selon l'une des revendications 12 à 15,
**caractérisé en ce que** l'élément de limitation (30) est relié au matériau en feuille (10) par une liaison par coopération de matière.
